(19) Europäisches Patentamt / European Patent Office / Office européen des brevets

(11) **EP 4 609 705 A1**

(12) **EUROPEAN PATENT APPLICATION**
published in accordance with Art. 153(4) EPC

(43) Date of publication:
**03.09.2025 Bulletin 2025/36**

(21) Application number: 23882731.5

(22) Date of filing: 26.10.2023

(51) International Patent Classification (IPC):
**A01H 6/20** (2018.01)    **A01H 5/00** (2018.01)
**A01H 5/10** (2018.01)    **A23L 19/00** (2016.01)

(52) Cooperative Patent Classification (CPC):
**A01H 5/00; A01H 5/10; A01H 6/20; A23L 19/00**

(86) International application number:
**PCT/JP2023/038753**

(87) International publication number:
**WO 2024/090529 (02.05.2024 Gazette 2024/18)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 26.10.2022 JP 2022171660

(71) Applicant: **Sakata Seed Corporation**
**Yokohama-shi**
**Kanagawa 224-0041 (JP)**

(72) Inventor: **FUNABIKI, Tomoaki**
**Yokohama-shi, Kanagawa 224-0041 (JP)**

(74) Representative: **Bandpay & Greuter**
**11, rue Christophe Colomb**
**75008 Paris (FR)**

(54) **BROCCOLI PLANT THAT PRODUCES SMALL FLOWER BUDS WITH UNIFORM SIZE AND HIGH YIELD, AND METHOD FOR PRODUCING PROCESSED PRODUCT THEREOF**

(57) A broccoli plant is disclosed that allows for production of florets with uniform size and high yield, and is suitable for a processed broccoli product that is obtained by dividing the broccoli plant into florets. This broccoli plant has genes involved in floret production in the nuclear genome and allows for production of florets with uniform size and high yield. The broccoli plant facilitates floret processing and makes it possible to provide a broccoli product with high commercial value that is obtained by dividing the broccoli plant into florets.

Fig. 1

**Description**

Technical Field

The present invention relates to a broccoli plant producing florets of uniform size with high yield, and a method for producing a processed product of florets from the broccoli plant.

Background Art

Brassicaceae plants are plant species originated from the Middle East and the Mediterranean coast, and plants of the genus Brassica include extremely important crops in agriculture. Among them, Brassica oleracea L. is a very significant plant species including Brassica oleracea L. var. capitata (cabbage), B.oleracea L. var. italica (broccoli), B.oleracea L. var. botrytis (cauliflower), B.oleracea L. var. gemmifera (Brussels sprout), B.oleracea L. var. gongylodes (kohlrabi), B.oleracea L. var. acephala (ornamental kale, kale), and B.oleracea L. var. alboglabra (Chinese kale).

Broccoli, which is a plant belonging to the genus Brassica of the family Brassicaceae, is popular with consumers because the plant is considered to be good for health since it is a green-yellow vegetable not only rich in vitamin B, vitamin C, and vitamin A but also contains dietary fiber, carotene and iron, and also because it can be used in a wide variety of dishes and cooked easily. On the other hand, broccoli is a popular crop among growers because; burden on shipping work is reduced as it is a lightweight vegetable; it can be cultivated in an open field so, the initial investment is relatively low and easily started; and a stable yield and sales price can be expected as the growth period is short.

Plant varieties are generally classified into open pollinated varieties and the first filial generation (hereinafter, referred to as "F1") varieties and F1 varieties are widespread in many crops. Broccoli, which is used for food, was mainly made up of open pollinated varieties until the 1960s, but the shift to F1 varieties progressed rapidly from the 1970s to the 1980s, so currently most broccoli varieties are F1 hybrid.

A broccoli head is required to have the following characteristics: a dense dome shape having beads (non-flowering broccoli buds) uniform and small in size, a deep and uniform green color, and good firmness, and the broccoli heads with these characteristics are traded on the market. On the other hand, since broccoli is ultimately separated into florets when cooked and eaten, sometimes it is more convenient to sell broccoli in the form of appropriately sized florets from the beginning. In fact, the distribution of broccoli products separated into florets is increasing, and due to their ease of use, high demand is expected to continue in the future. From such a background, if a broccoli plant with a large number of uniform-sized florets and excellent floret processing suitability can be bred, it is advantageous because it would be possible to provide broccoli florets of high commercial value.

A broccoli plant suitable for a broccoli product containing divided florets is desired to have, e.g., high yield of florets, florets uniform in size and a dark green color, as well as a head that can be easily separated and processed after harvest.

WO2007/062009 (PLT 1) proposes broccoli suitable for floret separation and processing. The broccoli disclosed has at least 50% of the florets on a whole head, which do not touch another floret on the same whole head and has extended secondary stems which support detached florets. This shape is considered to improve the quality of florets and make processing thereof facilitated.

WO2012/084702 (PLT 2) discloses a broccoli plant characterized in that florets are comprised within a plane substantially parallel to the ground. This shape is such that mechanical harvesting and processing are facilitated.

TAKAHASHI proposes in "Promising large head production for processing and industrial use of broccoli" (Vegetable Information, February, 2021, page 40 (NPT 1)) and "Technology for increasing floret yield by increasing the size of broccoli head" (NPL 2), an increase in the yield of florets by increasing the size of the head without adhering to conventional vegetable shipping standards, for reducing the production cost, which is a problem to be solved for realizing domestic broccoli for industrial processing use. In addition, Grandome is grown as a variety capable of maintaining the quality of florets even when the size of head is increased which effectively increases the yield.

Companies which are selling floret processed product have differentiated their products by lining up a wide variety of products having florets uniform in size (for example, HP of Ecofroz S.A, NPL 3). However, there is no disclosure of a specific broccoli plant producing florets of uniform size with high yield.

As described above, although broccoli plants that facilitate processing of florets of broccoli are proposed, there remains a need for a broccoli plant producing florets of uniform size with high yield, making separation and processing of florets easier after harvest.

Citation List

Patent Literature

PTL 1: WO2007/062009 A
PLT 2: WO2012/084702 A

Non Patent Literature

NPL 1: Megumu TAKAHASHI, "Large Head Production Promising for Processing and Industrial Use of Broccoli", Vegetable Information, February 2021, page 40.
NPL 2: NARO HP, "Technology for increasing floret yield by increasing the size of broccoli head", https://www.naro.go.jp/project/results/4th_laboratory/nivfs/2020/nivfs20_s01.html
NPL 3: Ecofroz HP https://www.ecofroz.com/

Summary of Invention

Technical Problem

The present inventor has found a novel broccoli plant having a genetic trait of providing production of florets of uniform size with high yield while having favorable broccoli traits. The present invention is based on the findings.

Solution to Problem

An object of the present invention is to provide a broccoli plant that enables the production of florets of uniform size with high yield, a broccoli plant producing florets of uniform size with high yield, and a method for producing a processed product of florets from the broccoli plant.
The broccoli plant according to the present invention is a broccoli plant or a progeny thereof, comprising in its nuclear genome a gene involved in producing florets, which enables production of florets of uniform size with high yield.
The method for producing a processed product of florets of a broccoli plant according to the present invention comprises the steps of:

providing the broccoli plant according to the present invention,
removing a secondary stem floret from a whole head of the broccoli plant, and
optionally packaging the florets of the secondary stems cut out.

Results of Invention

According to the present invention, a broccoli plant producing florets of uniform size with high yield while having favorable broccoli traits is provided. By using the broccoli plant of the present invention, it is possible to facilitate processing of florets of broccoli and provide a broccoli product containing divided florets having a high commercial value. By using as a parental line the broccoli plant according to the present invention, it is possible to breed a broccoli line producing florets of uniform size with high yield.

Brief Description of Drawings

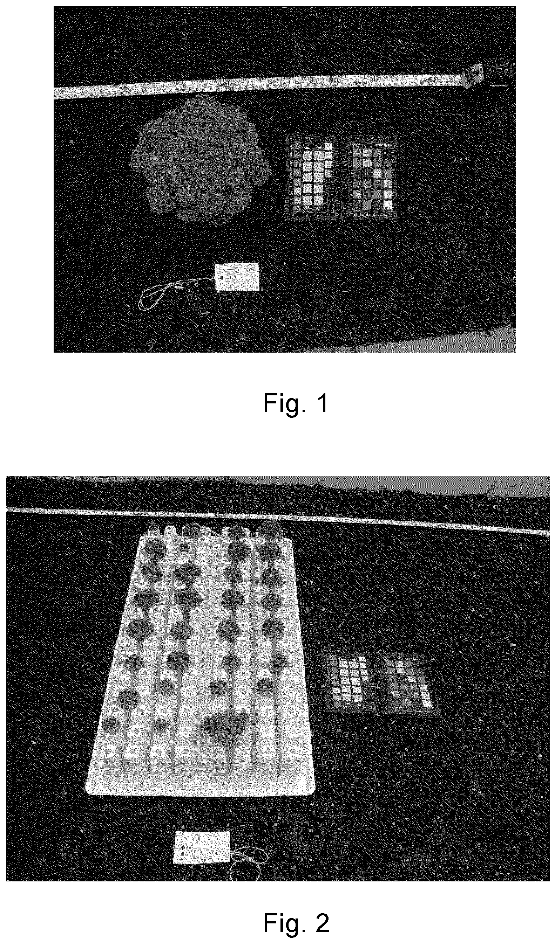

Fig. 1 is a photograph of broccoli line H, obtained in Example 3 and producing florets of uniform size with high yield, as viewed from above the top of the head.
Fig. 2 is a photograph showing florets of broccoli line H producing florets of uniform size with high yield, obtained in Example 3.

Description of Embodiments

Deposited seeds

The following seeds, identified as Deposits 1 to 3, have been deposited in connection with the present invention.

Deposit 1

Accession No.: FERM BP-22456
Indication for identification: SSC-BRO-22-001
Date of Deposit: August 1, 2022

Deposit 2

Accession No.: FERM BP-22457
Indication for identification: SSC-BRO-22-002
Date of Deposit: August 1, 2022

Deposit 3

Accession No.: FERM BP-22458
Indication for identification: SSC-BRO-22-003
Date of Deposit: August 1, 2022
Depositary (all deposits 1 to 3)
National Institute of Technology and Evaluation International Patent Organism Depositary Kazusakamatari
2-5-8, Kisarazu-shi, Chiba

Definitions

In the present invention and the present specification, "broccoli" refers to broccoli of Brassica oleracea L. var. italica; "Chinese kale" refers to Chinese kale of Brassica oleracea L. var. alboglabra; and "cauliflower" refers to cauliflower of Brassica oleracea L. var. botrytis. A plant obtained by the crossing of broccoli, Chinese kale, or cauliflower may be sometimes simply referred to as a Brassica line.
In the present invention and the present specification, a "whole head" refers to an assembly of florets.
In the present invention and the present specification, a "whole head" includes not only the whole head but also a part thereof. The term "whole head" is sometimes referred to simply as "head".
In the present invention and the present specification, a "floret" means a group of non-flowering broccoli floral buds and a portion of a stem(s) other than the main stem, such as a secondary and tertiary stem, etc., branched from the main stem and supporting the group which come together to comprise a whole head, optionally in contact with each other. As to the stem portions herein, a stem branched from the main stem is referred to as a secondary stem, and stems separated from the secondary stem to the extent that a single filament supports an individual flower bud is referred to as a tertiary stem, a quaternary stem, or the like.
In the present invention and the present specification, a "floret portion" refers to the florets which consist of a group of non-flowering broccoli flower buds and the secondary stem as a shaft supporting thereof wherein the secondary stem can be branched into tertiary stems, quaternary stems or the like. That is, the "floret portion" in the present invention refers to a "floret" supported by a secondary stem directly branched from a main stem, and refers to a "floret" including all the groups of floral buds present at the tip of stems such as a tertiary stem and a quaternary stem as long as they are supported by the secondary stem. Therefore, a "floret portion" is sometimes referred to as a "floret of a secondary stem".
Note that, in the present invention, the length of a stem supporting a floret portion can be adjusted by cutting or trimming. According to an embodiment of the present invention, it is preferable that the length from the tip of a floret to the tip of the stem is adjusted to be equal to the diameter of the floret. In contrast, florets prepared by controlling diameter or shape by transversely, longitudinally or obliquely separating, cutting or scraping a floral-bud group are not included in the definition of "floret portions" of the present invention. In the present invention, the shape of florets obtained by cultivation is evaluated whether it satisfies the numerical conditions of the "ratio of floret portion" of the present invention described below.
In the present invention and the present specification, the "diameter of a whole head" refers to either one of the values: the numerical value calculated by dividing the sum of a value measured as the minor-axis length and a value measured as the major-axis length when the whole head is viewed from the top by 2, or the value of a width when the whole head is viewed from the side direction.
In the present invention and the present specification, the "diameter of a floret" refers to either one of the values:

the numerical value calculated by dividing the sum of a value measured as the minor-axis length and a value measured as the major-axis length when the floret is viewed from the top by 2, or the value of the maximum width when the floret is viewed from the side direction.. Therefore, in the present invention, the "diameter of a floret portion" similarly refers to either one of the values: the numerical value obtained by dividing the sum of the value measured as the minor-axis length, the value measured as the major-axis length when the "floret portion" is viewed from the top by 2 or the value of the maximum width when the "floret portion" is viewed from the side direction. This is because, according to a preferred embodiment of the present invention, the floret of the broccoli plant according to the present invention, especially the "floret portion" at its widest point looks to form a nearly perfect circle when viewed from above.

In the present invention and the present specification, "the diameter of a whole head or a floret" is measured "at the time of harvest". As a mode for the measurement, the diameter may be measured directly by a measuring tool and/or further through the analysis of a photographed image. For example, the top or the side surface of a floret of broccoli may be photographed. A line for distinguishing the entire region of the floret from the background is drawn on the photographed image using a paint tool (on a personal computer or a tablet, and subsequently, the diameter of each floret may be measured using image-analysis software (such as ImageJ) in the processed image having the boundary line drawn therein. Also, the diameter of the whole head can be measured in a similar manner.

In the present invention and the present specification, florets "touch" means that the outer edge of the floret is in contact with another floret in a whole head when the whole head is viewed from above the top of the head.

In the present invention and the present specification, a "part of a plant body" includes cells or tissues of the plant body. Examples thereof include fruits, seeds, flowers, pollen, anthers, leaves, stems, roots, embryos, hypocotyls, meristem cells, and calluses. Other than the above, protoplasts obtained from cells of the plant body are also included.

Broccoli plant according to the present invention

The broccoli plant according to the present invention is a broccoli plant having in its nuclear genome a gene involved in producing florets, which enables production of florets of uniform size with high yield.

In this disclosure, "production of florets of uniform size with high yield" means that, when the diameter of the whole head is 14.0 cm or more and 17.0 cm or less, the ratio (%) of the number of floret portions with a diameter of more than 2.0 cm and 4.0 cm or less to the total number of floret portions, which is represented by the equation:

([Floret portions (number) of more than 2.0 cm and 4.0 cm or less in diameter /total floret portions (number)] $\times$ 100)

is at least 40%. In the following description, "when the diameter of a whole head is 14.0 cm or more and 17.0 cm or less, the ratio (%) of the number of floret portions of more than 2.0 cm and 4.0 cm or less in diameter to the total number of floret portions" may be simply referred to as "the ratio of floret portions".

According to an embodiment of the present invention, "the ratio of floret portions" is preferably 41% or more, 42% or more, 43% or more, or 44% or more. In a most preferred embodiment, "the ratio of floret portions" is 55% or more, 60% or more, 65% or more, or 70% or more.

When the diameter of the whole head is 14.0 cm or more and 17.0 cm or less, the number of floret portions of more than 2.0 cm and 4.0 cm or less in diameter is preferably 8 or more, and more preferably 9 or more, 10 or more, or 15 or more. Furthermore, in the broccoli plant according to the present invention, the number of floret portions of more than 2.0 cm and 4.0 cm or less in diameter is preferably more than 20 and more preferably more than 22.

According to a preferred embodiment of the present invention, the whole head of the broccoli plant, according to the present invention, is at the proper time of harvesting when the diameter of the whole head is 14.0 cm or more and 17.0 cm or less.

In addition, the broccoli plant according to the present invention can be defined by the weight thereof as an index in place of the number of floret portions. That is, a total weight of the floret portion of more than 2.0 cm and 4.0 cm or less is preferably 65 g or more, more preferably 90 g or more, and still more preferably 100 g or more.

In the broccoli plant according to the present invention, more than 50% of the florets on a whole head are touching another floret on the same whole head. According to a preferred embodiment, in the broccoli plant according to the present invention, all of the floret portions constituting an apical floret are touching each other. In addition, according to a preferred embodiment, in the broccoli plant according to the present invention, the floret portions constituting lateral florets are not in contact with each other.

In the broccoli plant according to the present invention, the longitudinal size of the whole head is preferably about 20 cm from the top of the whole head. Therefore, in the broccoli plant according to the present invention, the main stem of the whole head can be equally cut to a length of 25 cm from the top of the whole head, preferably 22 cm, and more preferably 20 cm.

The broccoli plant according to the present invention has a gene enabling "production of florets of uniform size with high yield" as described above, as the gene involved in producing florets in its nuclear genome. Therefore, the broccoli plant according to the present invention includes a broccoli plant having the gene in its nuclear genome and florets of uniform size and expresses the genetic trait thereof, as well as a broccoli plant having such a gene but is not expressed and the genetic trait will be expressed in its progeny.

In the present invention and the present specification, "having in its nuclear genome a gene involved in producing florets, which enables production of florets of uniform size with high yield" is sometimes expressed as "having a genetic trait of providing production of florets of uniform size with high yield" or "having a trait of producing florets of uniform size with high yield".

The broccoli plant according to the present invention has a trait suitable for processing florets, specifically a trait of producing florets of uniform size with high yield. As a broccoli floret product, a product having florets of 2 cm to 4 cm in diameter is preferable since it is bite-size, easy to eat, and florets of this size are also preferable because they can be applied to various uses in cooking. For this reasons, the broccoli plant according to the present invention having a large amount of florets in this size range is suitable for the processing of florets.

In addition, the present invention is advantageous in appearance as compared to cases where the florets are cut or trimmed to adjust the diameter thereof to fall within 2 cm to 4 cm or the shape is processed and is also advantageous because not only deterioration from e.g., a cut site but also a risk of miscellaneous bacterial growth from a cut edge can be avoided. Furthermore, the present invention is advantageous in that disposal of florets hardly used as a desired product and other parts can be reduced.

In the broccoli plant according to the present invention, since "floret portions", that is, "florets of the secondary stems" are uniform in size, by cutting the florets in the secondary stem, florets of uniform size can be obtained. Such processing of the secondary stem is particularly desirable because it reduces the number of steps and shortens the processing time.

According to a preferred embodiment of the invention, the broccoli plant according to the present invention has traits other than the trait of providing production of florets of uniform size with high yield, particularly that are the same or very similar to those traits of a conventional broccoli plant, as traits of an agricultural product. Examples of the traits as an agricultural product include taste, the shape of a whole head, the shape and color of a floret, qualities such as anthocyanin expression levels, and easiness in cultivation. Therefore, according to a preferred embodiment of the present invention, the broccoli plant according to the present invention has qualities such as a dense dome shape with fine uniform beads and a vivid dark green color of florets similarly to a whole head. A product having a high additional value can be provided if the florets uniform in diameter size are packed. In particular, in the present invention, broccoli plants with suppressed anthocyanin expression is preferable.

Progeny of broccoli plant according to the present invention

The broccoli plant according to the present invention is obtained by introducing a "gene of providing production of florets of uniform size with high yield", i.e., a "genetic trait of producing florets of uniform size with high yield", into a broccoli plant. Such a gene or genetic trait can be introduced, for example, by introducing a "gene involved in production of floret" which a broccoli plant producing florets of uniform size with high yield has, into the nuclear genome of a broccoli plant. The gene in the nuclear genome of the broccoli plant according to the present invention may be homozygous or heterozygous.

According to an embodiment of the present invention, the broccoli plant according to the present invention may be originated from an "intraspecific hybrid plant" obtained by crossing broccoli to broccoli, Chinese kale, or cauliflower. Therefore, in this embodiment, the broccoli plant according to the present invention is a plant individual to which a chromosome having a "gene enabling production of florets of uniform size with high yield" is inherited. In this disclosure, the term "chromosome" includes not only the entire chromosome but also a part thereof.

The broccoli plant according to the present invention may be an "interspecific hybrid plant". Examples of an interspecific hybrid plants include not only a plant produced by crossing between different species within the genus Brassica, but also a somatic hybrid plant obtained by cell fusion between different species within the genus Brassica, and a grafted hybrid plant obtained by grafting between different species within the genus Brassica.

In addition, the "plants originated from an intraspecific hybrid plant of broccoli and a plant of the genus Brassica except broccoli" include an intraspecific hybrid plant and a progeny thereof. Even in a progeny individual to which a "gene enabling production of florets of uniform size with high yield" originated from a plant of the genus Brassica is inherited, a "genetic trait of providing production of florets of uniform size with high yield" can be expressed by the gene.

In addition, "a progeny of the broccoli plant according to the present invention" includes a progeny obtained by an intraspecific crossing of the broccoli plant according to the present invention, and additionally, an individual obtained by using the broccoli plant according to the present invention as a parental line and a progeny thereof; a somatic hybrid plant obtained by cell fusion of a cell of the broccoli plant according to the present invention and another plant cell and a progeny thereof; and an individual obtained by grafting using the broccoli plant as a rootstock or scion and a progeny thereof. In this disclosure, the "progeny" includes both an individual obtained by intraspecific hybridization and an individual obtained by interspecies hybridization. In addition, the "individual obtained (using a plant) as a parental line" refers to an individual obtained by intraspecific hybridization, interspecies hybridization, cell fusion, or grafting using the plant as a parental line.

According to an embodiment of the present invention, the introduction of the "gene enabling production of florets of uniform size with high yield" into genomic DNA can be performed by, e.g., a crossbreeding method using broccoli and a plant of the genus Brassica having a trait of producing florets of uniform size with high yield as a parental line, or a genetic modification method such as genome editing.

The "genetic trait of providing production of florets of uniform size with high yield" in the broccoli plant according to the present invention shows recessive expression and can be genetically fixed. Therefore, when the broccoli plant according to the present invention is subjected to interspecific crossing or intraspecific crossing as a parental line, it is possible to develop a novel line of broccoli producing florets of uniform size with high yield.

F1 obtained by crossing the broccoli plant according to the present invention as parental lines exhibits positive overdominant or incomplete dominant expression, and can further express a trait of producing florets of uniform size with high yield.

Specific examples of the broccoli plant according to the present invention are plants identified by the accession numbers of deposited seeds, i.e., FERM BP-22456 (SSC-BRO-22-001), FERM BP-22457 (SSC-BRO-22-002) and FERM BP-22458 (SSC-BRO-22-003). The broccoli plant according to the present invention includes the following embodiments based on the specific plants deposited.

That is, the present invention also includes broccoli plants which have the "gene enabling production of florets of uniform size with high yield" or "a genetic trait of providing production of florets of uniform size with high yield" originated from the deposited plant.

In this disclosure, in the present invention and the present specification, "comprise" is synonymous with "have".

A broccoli plant having "a gene enabling production of florets of uniform size with high yield" or "a genetic trait of providing production of florets of uniform size with high yield" identified or represented by the deposited plant is also included in the present invention.

In the present invention and the present specification, a gene or a genetic trait "specified" or "represented" by a deposited plant means that a gene having an equivalent function, even if the function is not completely identical to that of the gene present in the deposited plant, is included.

A broccoli plant having a "gene enabling production of florets of uniform size with high yield" or "a genetic trait of providing production of florets of uniform size with high yield" which is to be found in the deposited plant is also included in the present invention.

In the present invention and the present specification, the gene or genetic trait "able to be found (be foundable)" in the deposited plant means that a gene having an equivalent function even if the function is not completely identical to that of the gene "found (be found)" in the deposited plant, is included.

In addition, the present invention includes a hybrid plant obtained by using a broccoli plant producing florets of uniform size with high yield and identified by accession number FERM BP-22456, accession number FERM BP-22457, or accession number FERM BP-22458, as a parental line, or a progeny thereof, that is, a broccoli plant producing florets of uniform size with high yield.

Method for producing broccoli plant according to the present invention

The production of the broccoli plant according to the present invention can preferably be performed by crossing broccoli to a plant obtained by crossing broccoli, Chinese kale or cauliflower, and developing a broccoli plant having a high ratio of floret portions from a progeny of the obtained seed based on the ratio as an index.

In the present invention and the present specification, the "production method" can also be rephrased as a "generation method", a "developing method", a "growth method", or a "breeding method". That is, the terms "production", "generation", "development", "growth", and "breeding" used herein are interchangeably used.

In the method for producing broccoli according to the present invention, the broccoli used as a parental line is not particularly limited but is preferably a variety cultivated as a crop. The varieties of broccoli currently distributed are mainly a single-head type and a stick broccoli. The single-head type broccoli refers to broccoli from which a single whole head having a diameter of up to about 12.0 cm is harvested but may be broccoli from which two or three or more whole heads having the same size are harvested. It has been reported that a single-head type variety is acceptable as a commercial product as long as a whole head has a diameter of up to about 20.0 cm depending on the use. The broccoli used as the parental line in the present invention is preferably an individual of the broccoli line handled as a single-head type broccoli in trading in Japan. The broccoli used as the parental line in the present invention is more preferably broccoli in which an outer edge of a floret is in contact with another floret within the same whole head when the whole head is viewed from above the top. By using a single-head type broccoli line, it is easier to develop a novel broccoli plant having a trait of producing florets of uniform size with high yield.

A preferred method for producing a broccoli plant according to the present invention includes, for example, a step of crossing broccoli to a plant obtained by crossing broccoli, Chinese kale or cauliflower to produce F1 seeds; a step of cultivating and selecting the plants from the F1 seeds based on the yield and size (uniform and small) of florets as an index to produce seeds; a step of performing self-pollination or back-crossing of the seed obtained from the selected plant and thereafter repeating self-pollination to develop a broccoli plant producing florets of uniform size with high yield; a step of back-crossing the broccoli plant producing florets of uniform size with high yield used as a recurrent parental line to a cytoplasmic male sterile line; and a step of crossing the developed broccoli plants producing florets of uniform size with high yield to each other. The number of repetitions of self-pollination and back-crossing is not particularly limited as long as it is 1 or more, 2 to 3 or 3 or more. During these steps, back-crossing, self-pollination, and cultivation can be performed in accordance with common methods of broccoli cultivation.

Production of F1 seeds of broccoli plant according to the present invention

According to an embodiment of the present invention, the broccoli plant according to the present invention is provided as F1. The method for producing F1 seeds of a broccoli plant according to the present invention includes a step of crossing the broccoli plant according to the present invention to a broccoli plant according to the present invention, or a broccoli plant except the broccoli plant according to the present invention, preferably with a broccoli plant of another line having a genetic trait of providing production of florets of uniform size with high yield; and a step of producing F1 seeds from individuals obtained by the crossing.

Since the "genetic trait of providing production of florets of uniform size with high yield" in the broccoli plant according to the present invention is of recessive expression, F1 seeds from which whole heads having a trait of producing the florets of uniform size with high yield can be harvested are obtained by intraspecific crossing to another broccoli line having the trait or by interspecific crossing with Brassica plant producing florets of uniform size with high yield. Hybridization and seed production can be performed by a conventional method.

According to an embodiment of the present invention, the F1 line obtained by crossing the broccoli plants according to the present invention as parental lines expresses a particularly preferred trait. Specifically, combinations described later in the Examples are mentioned. That is, as the broccoli plant used as the parental line, the broccoli lines A, E, K, and M can be used. As the combination of F1, a hybrid combination of line K or line CMS K and line E is preferable, and a hybrid combination of line A or line CMS A and line E is further preferable. It is also possible to use a broccoli plant, which produces florets of uniform size with high yield and is produced by the method for producing a broccoli plant according to the present invention.

The degree of the dominant effect of a desired trait in F1 is expressed by a potency ratio (Takeda, 1993), which can be calculated using the following equation modified by Ohgata et al. (2003) to distinguish positive and negative expression directions.

$$\text{Potency ratio} = 2 \times (F1-MP)/|P1-P2|$$

F1: Measured value of F1
P1, P2: Measured values of respective parental lines
MP (Intermediate parent): Average of parental lines

The potency ratio (h/d) is a quantitative measure indicating the degree of dominant effect of a quantitative gene. The degree of dominant effect can be estimated as follows: if $|h/d| = 1$, complete dominance; if $|h/d| = 0$, no dominance; if $0 < |h/d| < 1$, incomplete dominance; and if $1 < |h/d|$, overdominance (Takeda, 1983).

Use of broccoli plant according to the present invention: breeding material

The broccoli plant according to the present invention can be used, for example, as a parental line, that is, as a breeding material, for developing a novel broccoli plant that produces florets of uniform size with high yield. Furthermore, a hybrid plant obtained using the broccoli plant according to the present invention as a parental line, and a progeny thereof can be similarly used as a parental line for developing a novel broccoli plant producing florets of uniform size with high yield.

Specifically, a Brassica plant expressing a genetic trait of providing production of florets of uniform size with high yield can be produced, for example, by recurrent back-crossing of any Brassica plant or an interspecific hybrid plant originated from a Brassica plant to the broccoli plant according to the present invention. Also, a broccoli plant expressing a genetic trait of providing production of florets of uniform size with high yield can be produced by recurrent back-crossing any broccoli plant or an interspecific hybrid plant originated from a broccoli plant to the broccoli plant according to the present invention.

Method for producing broccoli's floret processed product according to the present invention

According to the present invention, a method for producing a processed product of broccoli florets is provided. The method includes a step of preparing the broccoli plant according to the present invention; a step of cutting out florets of secondary stems from a whole head of the broccoli plant; and a step of optionally packaging the florets of secondary stems cut out.

As described above, the broccoli plant according to the present invention has a trait of producing florets of uniform size with high yield. According to a preferred embodiment, the broccoli plant according to the present invention has a "ratio of floret portions" of at least 40% or more. Therefore, the "florets of the secondary stems" are uniform in size and high in yield. When florets in the secondary stems are cut off from a whole head of the broccoli plant according to the present invention, a large number of florets uniform in size can be obtained. According to an embodiment of the present invention, it is preferable to adjust the length from the floret tip to the stem tip to be the same as the diameter of the florets. According to a particularly preferred embodiment, when the diameter of the head is 14.0 cm or more and 17.0 cm or less, florets of secondary stems are cut out from the head of the broccoli plant in which the ratio (%) of the number of floret portions of more than 2.0 cm and 4.0 cm or less in diameter to the total number of floret portions (ratio of floret portions) is at least 40%.

The cut florets of the secondary stems are then appropriately placed in, e.g., a container or bag as a processed product, and if necessary, refrigerated or frozen. In the method according to the present invention, since florets uniform in size are obtained by cutting the florets in the secondary stems, benefits of reducing the number of processing steps and the working time can be obtained. The processed product of florets of broccoli obtained by the method according to the present invention has florets of 2 cm to 4 cm in diameter. The florets of this size are bite-size easy to eat, and can be applied to various uses in cooking. According to the method of the present invention, it is possible to provide a broccoli processed product having a high commercial value.

[Examples]

The present invention will be described in more detail with reference to Examples, but the present invention is not limited to the following Examples:

Example 1: Development of broccoli line A

Two broccoli lines having a large number of side branches, a downy mildew-resistant broccoli line, a Chinese kale line, and a cauliflower line (all possessed by SAKATA SEED CORPORATION) were provided and crossed, and then, among the progeny, Brassica line B, which has extended flower stems and relatively uniform and firm florets, was selected. This line was crossed with a semi-dome type broccoli line C having dark-color florets (possessed by SAKATA SEED CORPORATION) and then, from F1 (line D) thus obtained, selection based on indexes: sufficient yield and florets uniform in size, and small, and self-pollination were repeated. By the process, broccoli line A having a trait of providing formation of florets uniform in size was developed.

In the second year, line B as a seed parent was crossed with line C as a pollen parent and then F1 thus obtained was evaluated and after confirming that the florets were uniform in size, self-pollination was performed.

In the third year, 32 individuals of the F2 generation obtained were cultivated. While individuals having florets non-uniform in size or loosened heads in an early stage and individuals lacking in field holding ability were frequently found, a single individual having firm florets uniform and small in size was selected and self-pollinated.

In the fourth year, 40 individuals of the F3 generation were cultivated and 3 individuals having a trait of providing florets uniform and small in size and firm were selected and self-pollinated in the same manner as the previous year.

In the fifth year, 40 individuals of the F4 generation obtained respectively from the 3 individuals selected in the previous year were cultivated. From a population originated from one of the lines, three individuals having a trait of providing firm florets, uniform and small in size, and also having suppressed expression of anthocyanin in heads were selected and self-pollinated in the same manner as the previous year and the year before.

Thereafter, sowing, selection, and self-pollination were continuously repeated in the same manner. In the tenth year, seeds were produced from an individual having good seed productivity and sown. As a result, a plant body producing florets uniform in size was successfully confirmed. It was also confirmed that anthocyanin expression in florets was suppressed.

A cytoplasmic male sterile line (possessed by SAKATA SEED CORPORATION) was backcrossed to line A eight times to develop CMS A of a cytoplasmic male sterile line having the same nuclear gene as in line A.

Example 2: Development of broccoli line E

With a broccoli line (line F, possessed by SAKATA SEED CORPORATION) having florets in large number and being extremely firm, the Brassica line B having extended flower stems and florets relatively uniform in size and firm was crossed and self-pollinated to develop, from the F1 thus obtained, broccoli line E producing florets of uniform size with high yield.

The F1 obtained by crossing broccoli line F as a seed parent with Brassica line B as a pollen parent was evaluated and after confirming that the F1 had florets uniform in size and extremely firm, self-pollination was performed. Then, 69 individuals of F2 generation obtained were grown. While individuals having florets not uniform in size or malformed in shape of entire heads were observed, four individuals having florets uniform in size and firm were selected, and self-pollinated.

In the following year, 25 individuals were grown from each of the selected four lines of the F3 generation and then evaluation was performed based on whether an individual has a trait of providing florets uniform in size and firm, in the same manner as the previous year. As a result, six individuals were selected from a population originated from a single line and self-pollinated. Of these selected individuals, two individuals had florets with suppressed anthocyanin expression.

Subsequently, 25 individuals were grown from each of the selected six lines of the F4 generation, and evaluation was performed. As a result, from the line originating from the individual having florets with suppressed anthocyanin expression, two individuals having the florets uniform in size and firm were selected, and self-pollinated. Thereafter, sowing, selection, and self-pollination were repeated continuously in the same manner and then, five years later, seeds were produced from individuals having good seed productivity and were sown. It was confirmed that the obtained plant body had uniform florets, and the total number of the florets was extremely excellent. It was also confirmed that the expression of anthocyanin in florets was suppressed.

Seeds of line E have been deposited at the depository under accession number FERM BP-22456 (indication for identification: SSC-BRO-22-001).

Example 3: Development of broccoli line G and line H

Crossing was performed using line A as a seed parent and the precursor line of line E having almost fixed main traits as a pollen parent by bud pollination, resulting in the development of line G (F1). In the following summer, when line G was sown and the phenotypic traits were checked, it was confirmed that florets were uniform in size, a total number of florets was superior compared to conventional F1, and the expression of anthocyanin in the heads was suppressed. In the following year, line E which had been further self-pollinated, was used as a pollen parent, and a trial of line G was performed. It was confirmed that the same traits as in the previous year were expressed.

Line CMS A was crossed as a seed parent to line E as a pollen parent to develop line H. In the trial with sowing in the summer of the same year, it was confirmed that florets of line H were uniform in size and excellent in total number, and the expression of anthocyanin was suppressed.

Seeds of line H have been deposited at the depository under accession number FERM BP-22457 (indication for identification: SSC-BRO-22-002).

Example 4: Development of Broccoli Line K

According to the same manner as in Examples 1 and 2, a line having a large number of florets was crossed to a line having elongated flower stems and florets relatively uniform in size to develop broccoli line I, providing florets uniform in size. However, it was confirmed that florets of line I lacked firmness and were deficient in yield compared to commercially available varieties. To overcome this deficiency in yield, line I was crossed with F1 line D, which was used to develop line A, to perform three-way crossing. The obtained seeds were sown, grown, selected and self-pollinated. This process was repeated to develop broccoli line K producing florets of uniform size with high yield and improved in yield of florets. In addition, it was confirmed that line K had florets with suppressed anthocyanin expression.

A cytoplasmic male sterile line possessed by SAKATA SEED CORPORATION was backcrossed to line K seven times to develop CMS K, a cytoplasmic male sterile line having the same nuclear gene as line K.

Example 5: Development of line L

Crossing was performed using line CMS K as a seed parent and line E as a pollen parent to develop line L. It was confirmed that the size of florets of line L was uniform, the total number of florets was superior to that of the conventional F1 variety, and expression of anthocyanin in the head was suppressed.

Seeds of line L have been deposited at the aforementioned depository under accession number FERM BP-22458 (indication for identification: SSC-BRO-22-003).

Example 6: Development of line M

By crossing a broccoli plant having good growth at low temperatures and suppressed anthocyanin expression (possessed by SAKATA SEED CORPORATION) to an extreme dome head type broccoli with very fine beads (possessed by SAKATA SEED CORPORATION), a progeny line was obtained, and then the line and a broccoli line having a slightly flat and elastic whole head (possessed by SAKATA SEED CORPORATION) were crossed to select and develop a line based on the characteristics of beads of a head, dark green although expression of anthocyanin was suppressed, good growth and a large number of florets as indexes. The resultant broccoli line was referred to as line M.

Although the breeding process and the breeding material of line M are different from those of Examples 1 to 5, based on the evaluation results described later, line M was found to be an example demonstrating that a broccoli plant producing florets of uniform size with high yield can be developed even if the genetic background of the broccoli is different from the lines of Examples 1 to 5.

Evaluation of broccoli plants

After sowing, seedlings were grown in cell trays and transplanted in a field at the suitable stage, and conventional cultivation management was performed. When whole heads reached the proper time of harvesting, florets were harvested and the measurement of the harvested product was performed. For the number of florets, an average value thereof was used. The measured florets were all florets of secondary stems.

[Test 11 Determination of ratio of floret portions in autumn slot test cultivation

The ratio of floret portions of six broccoli lines (lines A, E, H, K, L, and M) developed in Examples 1 to 6, four commercially available broccoli lines, and two broccoli lines (lines O and N) possessed by SAKATA SEED CORPORATION were calculated and compared.

The following commercially available broccoli lines were chosen: "Monflor" is an F1 hybrid sold by Syngenta, having the Separated Small Floret feature and said to be suitable for processing demand. It is a variety included in the Pinto database (see Patent Information and Transparency On-line) in Patent Literature 2 described above. "Skytree" is an F1 hybrid sold by Bayer, having the concept of Easy Floret feature, and is being sold for floret quality. "Ohayo" is one of the main representative F1 hybrid varieties in Japan, which is sold by SAKATA SEED CORPORATION and is a representative of an early maturing variety. In addition, "Grandome" is one of the main representative F1 hybrid varieties in Japan, sold by SAKATA SEED CORPORATION, and is a representative medium to late maturing variety, and also disclosed in Non-Patent Literature 1 and Non-Patent Literature 2 described above.

Similarly, broccoli lines O and line N (both possessed by SAKATA SEED CORPORATION) were evaluated at the same time, are representative of early maturing conventional broccoli having a slightly flat and good-quality head and representative of medium-late maturing conventional broccoli having a dome head shape and providing high yields of whole heads, respectively.

The cultivation site was the Kakegawa Research Center of SAKATA SEED CORPORATION, and the cultivation was performed in the typical autumn-slot cultivation period with sowing on August 10 and transplanting on September 7, during which the whole head formation time coincides with the drop in temperature. Then, during the proper time of harvesting a whole head, floret portions were measured. After the whole head was harvested in the field, it was measured directly using an electronic scale and a measuring tool. Further, floret portions were separated and similarly measured directly using an electronic scale and a measuring tool. The number of floret (florets in contact) portions in contact with each other and the number of floret (florets not in contact) portions not in contact with each other were obtained. From these, the contact ratio (%) of the florets was calculated. The results are as shown in Table 1. Under the present cultivation conditions, it was confirmed that the apical florets tend to be florets mutually in contact, and the lateral florets tend to be florets not mutually in contact.

[Table 1]

| Table 1 Measurement of Whole Head in Autumn Slot Test Cultivation | | | | | | | | | | | |
| --- | --- | --- | --- | --- | --- | --- | --- | --- | --- | --- | --- |
| | | | | | | | | | | | |
| Line Name | Number of individuals measured | Diameter [cm] of head | Weight [g] of head | Total number of floret portions | Total weight [g] of floret portions | Number of 2-4 cm floret portions | Ratio [%] of 2-4 cm floret portions | Total weight [g] of 2-4 cm floret portions | Number of florets in contact | Number of florets not in contact | Ratio [%] of florets in contact |
| Line H | 3 | 14.7 | 522.8 | 32.0 | 222.8 | 22.7 | 71.1% | 154.9 | 28.7 | 3.3 | 89.6% |
| Line L | 3 | 14.7 | 486.7 | 28.7 | 146.7 | 21.3 | 74.3% | 105.3 | 22.3 | 6.3 | 77.9% |
| Line A | 3 | 14.3 | 470.4 | 23.7 | 240.4 | 10.0 | 42.3% | 48.8 | 18.7 | 5.0 | 78.9% |
| Line K | 3 | 14.3 | 183.1 | 23.0 | 35.1 | 10.0 | 43.5% | 27.3 | 19.3 | 3.7 | 84.1% |
| Line E | 3 | 15.4 | 500.5 | 28.3 | 270.9 | 16.7 | 59.1% | 118.5 | 23.7 | 4.7 | 83.5% |
| Line M | 3 | 15.2 | 589.6 | 21.7 | 324.6 | 8.7 | 40.4% | 75.8 | 19.0 | 2.7 | 87.7% |
| Monflor | 3 | 14.0 | 299.8 | 24.3 | 142.5 | 9.0 | 37.1% | 39.0 | 19.7 | 4.7 | 80.8% |
| Skytree | 3 | 14.3 | 347.4 | 22.3 | 191.4 | 8.0 | 36.1% | 47.0 | 22.0 | 0.3 | 98.5% |
| Ohayo | 3 | 15.8 | 557.3 | 21.3 | 330.7 | 8.0 | 37.3% | 62.7 | 21.0 | 0.3 | 98.4% |
| Grandome | 3 | 16.1 | 604.7 | 20.0 | 352.7 | 7.0 | 35.0% | 56.0 | 20.0 | 0.0 | 100.0% |
| Line O | 3 | 14.3 | 450.4 | 20.0 | 250.4 | 6.3 | 31.9% | 29.3 | 15.7 | 4.3 | 78.3% |
| Line N | 3 | 14.7 | 486.2 | 23.0 | 276.2 | 7.0 | 30.9% | 27.6 | 20.0 | 3.0 | 87.0% |

In the table, the term "2-4cm floret portions" to "floret portions having a diameter of more than 2.0 cm and 4.0 cm or less".

As is apparent from the results of Table 1, the "ratio of floret portions" for lines A, E, H, K, L, and M developed in Examples 1 to 6 were all more than 40%. In contrast, the "ratio of floret portions" for commercially available varieties, Monflor, Skytree, Ohayo and Grandome were all in the 30% range. From the above, it was shown that the ratio of florets in the broccoli plant according to the present invention is clearly larger than those in conventionally known broccoli plants.

In addition, the total weights of floret portions having a diameter of more than 2.0 cm and 4.0 cm or less for lines E, H, L, and M were all 75.8 g or more. It was demonstrated that the total weight of them is large compared to the total weights (less than 62.8 g) of commercially available varieties, Monflor, Skytree, Ohayo and Grandome. The ratios of floret portions and the total weights of floret portions for both lines O and N were less than those of the commercially available varieties.

[Test 2] Measurement of the whole head in spring slot test cultivation

Whether a trait of providing florets of uniform size with high yield is expressed in a different period from that of Test 1 was examined. Lines H and L were compared to three commercially available broccoli lines, Skytree, Ohayo, and Grandome.

The cultivation site was the Kakegawa Research Center of SAKATA SEED CORPORATION, and the cultivation was performed in the typical spring-slot cultivation period with sowing on January 25 and transplanting on March 11, during which the whole head formation time coincides with the rising temperature. The measurement of floret portions at the proper time of harvesting a curd, calculation of the ratio of floret portions, and the ratio of the florets mutually in contact were performed in the same manner as in Test 1. The results are shown in Table 2.

[Table 2]

| Table 2 Measurement of Whole Head in Spring Slot Test Cultivation | | | | | | | | | | | |
|---|---|---|---|---|---|---|---|---|---|---|---|
| Line Name | Number of individuals measured | Diameter [cm] of head | Weight [g] of head | Total number of floret portoins | Total weight [g] of floret portions | Number of 2-4 cm floret portions | Ratio [%] of 2-4 cm floret portions | Total weight [g] of 2-4 cm floret portions | Number of florets in contact | Number of florets not in contact | Ratio [%] of florets in contact |
| Line H | 3 | 15.7 | 548.3 | 33.3 | 240.0 | 20.7 | 61.9% | 158.7 | 21.3 | 12.0 | 64.0% |
| Line L | 1 | 15.0 | 392.0 | 36.0 | 120.0 | 20.0 | 55.6% | 84.0 | 28.0 | 8.0 | 77.8% |
| Skytree | 2 | 16.5 | 350.0 | 15.0 | 202.0 | 5.5 | 36.7% | 26.0 | 14.0 | 1.0 | 93.3% |
| Ohayo | 2 | 15.0 | 299.0 | 22.5 | 134.0 | 8.0 | 35.5% | 36.5 | 19.5 | 3.0 | 86.7% |
| Grandome | 2 | 16.5 | 571.5 | 21.5 | 316.0 | 8.0 | 37.2% | 55.5 | 21.5 | 0.0 | 100.0% |

In the table, the term "2-4 cm floret portions" refers to "floret portions having a diameter of more than 2.0 cm and 4.0 cm or less".

In both lines H and L, the "ratios of floret portions" were 55.6% or more. In contrast, in the commercial varieties "Skytree", "Ohayo", and "Grandome", the "ratios of floret portions" were all less than 37.3%. In addition, the total weight of the floret portions having a diameter of more than 2.0 cm and 4.0 cm or less was less than 55.6 g in any one of "Skytree", "Ohayo", and "Grandome", but the total weight in each of lines H and line L, was 84.0 g or more and obviously heavy. As a result, it was confirmed that the broccoli plant according to the present invention can produce florets of uniform size with high yield no matter when the cultivation period is.

[Test 3] Verification of dominant effect in F1

With respect to lines H and L as F1 of the broccoli lines, according to the present invention, the potency ratio at the suitable time of whole head harvesting in autumn slot was calculated. The results are as shown in Table 3.

[Table 3]

| Table 3 Potency Ratio of F1 in Trait of Producing Small Florets Uniform in Size with High Yield in Autumn Slot Test Cultivation | | | | | |
|---|---|---|---|---|---|
| | | | | | |
| F1 | Parent | Parent | Ratio [%] of 2-4 cm floret portions | Total weight [g] of 2-4 cm floret portions | |
| Line H | Line A | Line E | 2.4 | 2.0 | |
| Line L | Line K | Line E | 2.9 | 0.7 | |
| | | | | | |
| | | | 1 | : Complete dominance | |
| | | | 0 | : Non-dominance | |
| | | | 0 to 1 | : Imcomplete dominance | |
| | | | 1< | : Overdominance | |

In the table, the term "2-4 cm floret portions" refers to "floret portions having a diameter of more than 2.0 cm and 4.0 cm or less".

The value of the potency ratio in the autumn slot for line H was as follows. In the case of the ratio of floret portions (more than 2.0 cm and 4.0 cm or less)/total floret portions (number), the value was 2.4. In the case of the ratio of floret portions (more than 2.0 cm and 4.0 cm or less in diameter)/total floret portions (total weight), the value was 2.0. In line H, the degree of dominant effect for any of the traits was estimated to be positive for overdominance. In contrast, those numerical values for line L were as follows. In the case of the ratio of floret portions (more than 2.0 cm and 4.0 cm or less)/total floret portions (number), the value was 2.9. In the case of the ratio of floret portions (more than 2.0 cm and 4.0 cm or less in diameter)/total floret portions (total weight), the value was 0.7. Thus, in line L, the degree of dominant effect in the case of the ratio of florets (more than 2.0 cm and 4.0 cm or less)/total florets (number) was positive overdominant, and in the case of the ratio of floret portions (more than 2.0 cm and 4.0 cm or less in diameter)/total floret portions (total weight) was positive incomplete dominant. It was demonstrated that line H or line L, which is F1 obtained by crossing a broccoli plant according to the present invention as both parental lines, the degree of the dominant effect of a trait of producing florets uniform size with high yield, is positive in overdominance or incomplete dominance, representing a more significant effect.

All publications, patents, and patent applications cited herein are hereby incorporated by reference in their entirety.

**Claims**

1. A broccoli plant or a progeny thereof, comprising in its nuclear genome a gene involved in producing florets, which enables production of florets of uniform size with high yield.

2. The broccoli plant or a progeny thereof according to claim 1, wherein the gene involved in producing florets is capable of expressing a genetic trait in which a ratio (%) of the number of floret portions of more than 2.0 cm and 4.0 cm or less in diameter to a total number of floret portions (the ratio of floret portions) is at least 40%, when the diameter of a whole head is 14.0 cm or more and 17.0 cm or less.

3. The broccoli plant according to claim 1 or 2, wherein expression of anthocyanin is suppressed.

4. The broccoli plant or a progeny thereof according to any one of claims 1 to 3, wherein the gene is originated from broccoli, Chinese kale, or cauliflower.

5. The broccoli plant or a progeny thereof according to any one of claims 2 to 4, wherein the gene involved in producing florets is capable of expressing a genetic trait in which the ratio of floret portions is 41% or more, 42% or more, 43% or more, or 44% or more.

6. The broccoli plant or a progeny thereof according to any one of claims 2 to 4, wherein the gene involved in producing florets is capable of expressing a genetic trait in which the ratio of floret portions is 55% or more, 60% or more, 65% or more, or 70% or more.

7. The broccoli plant or a progeny thereof according to any one of claims 1 to 6, comprising the genetic trait of providing production of florets of uniform size with high yield which is originated from a plant identified or represented by accession number FERM BP-22456, accession number FERM BP-22457, or accession number FERM BP-22458; or the genetic trait of providing production of florets of uniform size with high yield which is foundable in any one of the deposited plants.

8. The broccoli plant or a progeny thereof according to any one of claims 1 to 7, wherein the ratio (%) of the number of floret portions of more than 2.0 cm and 4.0 cm or less in diameter to a total number of floret portions (the ratio of floret portions) is at least 40%, when a diameter of a whole head is 14.0 cm or more and 17.0 cm or less.

9. The broccoli plant or a progeny thereof according to claim 8, wherein the ratio of floret portions is 41% or more, 42% or more, 43% or more, or 44% or more.

10. The broccoli plant or a progeny thereof according to claim 8, wherein the ratio of floret portions is 55% or more, 60% or more, 65% or more, or 70% or more.

11. The broccoli plant or a progeny thereof according to claim 8, deposited under an accession number FERM BP-22456, an accession number FERM BP-22457, or an accession number FERM BP-22458.

12. The broccoli plant or a progeny thereof according to any one of claims 1 to 11, wherein a total number of floret portions is 8 or more, when a diameter of a whole head is 14.0 cm or more and 17.0 cm or less.

13. The broccoli plant or a progeny thereof according to any one of claims 1 to 12, wherein a weight of a floret portion having a diameter of more than 2.0 cm and 4.0 cm or less is 65 g or more, when the diameter of a whole head is 14.0 cm or more and 17.0 cm or less.

14. The broccoli plant or a progeny thereof according to any one of claims 1 to 13, having more than 50% of florets on a whole head, which touch another floret on the same whole head.

15. A hybrid plant or a progeny thereof, obtained by using the broccoli plant according to any one of claims 1 to 14 as a parental line.

16. A part of a plant body of the broccoli plant according to any one of claims 1 to 15.

17. The broccoli plant according to claim 16, which is a head or a floret.

18. A seed of the broccoli plant according to any one of claims 1 to 15.

19. A method for producing an F1 seed of the broccoli plant according to any one of claims 1 to 15, the method comprising the steps of:

crossing the broccoli plant according to any one of claims 1 to 15 to the broccoli plant according to any one of claims 1 to 15 or to another plant which is capable of crossing to the broccoli plant according to any one of claims 1 to 15; and

harvesting an F1 seed from an individual obtained by the crossing.

20. A method for producing a processed product of florets of broccoli, the method comprising the steps of:

providing the broccoli plant according to claim 1 or 2;
cutting out florets of secondary stems from a whole head of the broccoli plant; and
optionally packaging the cut out florets of secondary stems.

21. The method according to claim 20, wherein the broccoli plant is the broccoli plant according to claim 8.

22. A method for producing a processed product of florets of broccoli, comprising: the steps of: cutting out florets of secondary stems from a head of a broccoli plant in which a ratio (%) of a number of floret portions of more than 2.0 cm and 4.0 cm or less in diameter to a total number of floret portions (the ratio of floret portions) is at least 40% when a diameter of the head is 14.0 cm or more and 17.0 cm or less; and optionally packaging the cut our florets of the secondary stems.

Fig. 1

Fig. 2

## INTERNATIONAL SEARCH REPORT

| International application No. |
|---|
| **PCT/JP2023/038753** |

| **A.** | **CLASSIFICATION OF SUBJECT MATTER** |
|---|---|

*A01H 6/20*(2018.01)i; *A01H 5/00*(2018.01)i; *A01H 5/10*(2018.01)i; *A23L 19/00*(2016.01)i
FI: A01H6/20; A01H5/10; A01H5/00 Z; A23L19/00 A

According to International Patent Classification (IPC) or to both national classification and IPC

| **B.** | **FIELDS SEARCHED** |
|---|---|

Minimum documentation searched (classification system followed by classification symbols)

    A01H6/20; A01H5/00; A01H5/10; A23L19/00

Documentation searched other than minimum documentation to the extent that such documents are included in the fields searched

    Published examined utility model applications of Japan 1922-1996
    Published unexamined utility model applications of Japan 1971-2023
    Registered utility model specifications of Japan 1996-2023
    Published registered utility model applications of Japan 1994-2023

Electronic data base consulted during the international search (name of data base and, where practicable, search terms used)

    JSTPlus/JMEDPlus/JST7580 (JDreamIII); CAplus/MEDLINE/EMBASE/BIOSIS (STN)

| **C.** | **DOCUMENTS CONSIDERED TO BE RELEVANT** | |
|---|---|---|

| Category* | Citation of document, with indication, where appropriate, of the relevant passages | Relevant to claim No. |
|---|---|---|
| X | JP 2022-032756 A (TAKII SHUBYO KK) 25 February 2022 (2022-02-25) | 1-7, 15-20 |
| | claims 1-8, paragraphs [0100]-[0104], example 1 | |
| Y | | 1-10, 12-22 |
| A | | 11 |
| X | JP 2014-501523 A (SYNGENTA PARTICIPATIONS AG) 23 January 2014 (2014-01-23) | 1-7, 15-20 |
| | claims 1-16, paragraphs [0048], [0061], example 3 | |
| Y | | 1-10, 12-22 |
| A | | 11 |
| X | SHAN, Fake et al. Effects of Vertical Smashing Rotary Tillage on Root Growth Characteristics and Yield of Broccoli. Agriculture. June 2022, vol. 12, article no. 928 | 1, 2, 4-7, 15-20 |
| | abstract, "3.5. Characteristics of Broccoli" of pp. 7-8 | |
| Y | | 1-10, 12-22 |
| A | | 11 |
| Y | JP 2009-516528 A (SEMINIS VEGETABLE SEEDS, INC.) 23 April 2009 (2009-04-23) | 1-10, 12-22 |
| | paragraph [0012] | |

✓ Further documents are listed in the continuation of Box C.     ✓ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 November 2023** | **12 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/038753**

**C.    DOCUMENTS CONSIDERED TO BE RELEVANT**

| Category* | Citation of document, with indication, where appropriate, of the relevant passages | Relevant to claim No. |
|---|---|---|
| Y | WO 2020/013190 A1 (SAKATA SEED CORPORATION) 16 January 2020 (2020-01-16)<br>paragraph [0021] | 1-10, 12-22 |
| A | | 11 |
| A | TAKAHASHI, Megumu et al. Harvesting Two Heads from One Stock of Broccoli (Brassica oleracea L. var. italica) 'Yumehibiki' by Pinching the Shoot Apical Bud in Autumn Cropping. The Horticulture Journal. 2019, vol. 88, no. 3, pp. 364-372<br>the whole document | 1-22 |
| A | TAKAHASHI, Megumu et al. Enlarging Broccoli (Brassica oleracea L. var. italica) Heads by Extending the Growing Period and Sparse Planting to Increase Floret Yield. The Horticulture Journal. 2021, vol. 90, no. 1, pp. 75-84<br>the whole document | 1-22 |
| A | CORDERO, Ma. de Lourdes Fraire et al. EFFECT OF VARIETIES AND PLANT DENSITY ON THE PHYSICAL QUALITY OF BROCCOLI (Brassica oleracea var. italica). Rev. Fitotec. Mex. 2010, vol. 33, no. 2, pp. 141-147<br>the whole document | 1-22 |

Form PCT/ISA/210 (second sheet) (January 2015)

## INTERNATIONAL SEARCH REPORT
### Information on patent family members

International application No.

**PCT/JP2023/038753**

| Patent document cited in search report | | | Publication date (day/month/year) | Patent family member(s) | | | Publication date (day/month/year) |
|---|---|---|---|---|---|---|---|
| JP | 2022-032756 | A | 25 February 2022 | US 2022/0046882 A1 claims, paragraphs [0160]-[0165], example 1 | | | |
| JP | 2014-501523 | A | 23 January 2014 | US 2013/0312140 A1 claims, paragraphs [0047], [0060], example 3 WO 2012/083526 A1 EP 2654408 A1 | | | |
| JP | 2009-516528 | A | 23 April 2009 | US 2007/0118935 A1 paragraph [0013] WO 2007/062009 A2 EP 2422616 A2 CN 101394735 A | | | |
| WO | 2020/013190 | A1 | 16 January 2020 | US 2021/0274732 A1 paragraph [0027] EP 3821703 A1 KR 10-2021-0019070 A CN 112566491 A | | | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007062009 A

- WO 2012084702 A

**Non-patent literature cited in the description**

- **MEGUMU TAKAHASHI**. Large Head Production Promising for Processing and Industrial Use of Broccoli. *Vegetable Information*, February 2021, 40